# EUROPEAN PATENT APPLICATION

(11) **EP 1 536 022 A1**
(43) Date of publication of application: **01.06.2005**
(21) Application number: 03783905.7
(22) Date of filing: 08.08.2003
(51) Int. Cl.: C12Q 1/68

(54) **METHOD FOR COMPARING GENE EXPRESSION LEVEL**

(30) Priority: 09.08.2002 CN 02138093
(71) Applicant: Zhou, Guohua, Nanjing, Jiangsu 210002 (CN); Huadong Research Institute for Medicine and Biotechnics, Nanjing, Jiangsu 210002 (CN)
(72) Inventor: ZHOU, Guohua, Nanjing, Jiangsu 210002 (CN)
(74) Representative: Viering, Jentschura & Partner
(86) International application number: PCT/CN2003/000648
(87) International publication number: WO 2004/015137

(57) **Abstract**

The inventive method for comparatively assaying gene expression level between various gene sources comprises step 1 of labeling the mRNA of a given gene from different sources, respectively, through a suitable labeling process; step 2 of mixing the labeled fragments in one receptacle as PCR templates; step 3 of performing PCR by using source-specific oligonucleotides and a gene-specific oligonucleotide as primers; and step 4 of detecting the sequence of the amplified DNA fragments by the method based on bioluminometric assay. The base sequence in the sequencing profile is related to the gene source, and the signal intensity of each base is related to the gene expression level from the corresponding source. The ratio of signal intensities accurately is proportional to the abundance of the transcript among the corresponding sources.

## Description

### BACKGROUND OF THE INVENTION

### 1. Field of the Invention

This invention relates to a method for quantitatively determining DNA fragment in a DNA mixture, which includes comparatively analyzing the expression level of a given gene in different sources.

### 2. Description of the Related Technology

With the progress of the molecular biology, the whole genomic DNA of several tens of biological species have been sequenced, and the human genome project has already been finished [Science, 291 (5507), 1304-51 (2001); Nature, 409 (6822), 934-41 (2001)]. The information is transcripted by mRNA to produce protein according to the information coded in the genome. As the structure of the whole human genome is clarified, the next step is to analyze gene functions coded in genomes. Gene function analysis includes the understanding of the distribution of mRNA, the transcription products of genes, in a cell or different organs in a body, as well as the function, the distribution and the amount of the expressed proteins by the corresponding genes. Comparative analysis of gene expression profiling is essential to find the new functions of genes and to clarify the act of interacting between genes or between proteins [Gene 135, 265-74 (1993)]. Therefore gene expression profiling is becoming one of the main topics in DNA analysis. In clinical medicine research, new genes related to diseases can be found by quantitatively comparing the gene expression levels of given genes between different sources, such as different individuals (e.g. healthy persons and patients) and different organs (e.g. heart, lung or brain). These disease-related genes are very helpful for screening disease-specific drug targets. In addition, comparative analysis of the expression level of disease-related genes in the related organs is very useful for the accurate disease diagnosis at early stage. Furthermore, gene expression profiling in different sources is helpful for biomolecular researchers to find new functional genes used for upgrading a biological species.

The analysis of the quantities of mRNAs in a cell or tissue is called as gene expression profiling. As mRNA is easily digested by ribonuclease (RNase H enzyme) which is in a cell, the analysis of mRNA is often carried out by using its complementary strand called cDNA (complementary DNA) which is produced by reverse transcription of mRNA by using a reverse transcriptase. Up-to-now, there are various methods used for gene expression profiling, such as Northern blotting [Proc. Natl. Acad. Sci. USA, 85, 5698-702 (1988)], real-time PCR [Anal. Biochem. 220, 384-90 (1994)], sequencing [Science, 270, 484-7 (1995); Methods in Molecular Biology, 99, 297-319 (2000)] and microarray [Science, 270, 467-70 (1995); Biotechniques, 29, 548-50, 552-4 (2000); Nature Biotechnology, 14, 1681-4 (1996)].

Northern blotting is a classical method which is mainly used for analyzing the expression level of several or tens of genes. The detection procedure is very complicated and the test operators should be well trained. Furthermore, it used radioactive substance which is harmful to the operators and environment. Also its sensitivity is not so high, and it is impossible to detect small amount of gene expression products.

Real-time PCR uses a gene-specific primer and a polyadenine nucleotide (polyA) primer to amplify cDNA fragments (transcripts of mRNA), and the amplified products are separated by electrophoresis. Gene expression information is obtained from the intensities of the bands in the electrophoretogram. As this method used PCR for the amplification of cDNA, the sensitivity is very high. However the quantification is not so satisfactory because the ratio between each of amplified fragments could not completely reflect the ratio between each of the templates before amplification.

Sequencing method is based on the large scale of cDNA sequencing, followed by the frequency calculation of each cDNA. A method was developed, called serial analysis of gene expression (SAGE). This method allows the quantitative and simultaneous analysis of a large number of transcripts. SAGE method can be used to systematically analyze transcripts present in a cell or tissue. At first, the beaded cDNAs are digested by a 4-base restriction cutter enzyme (usually *Nla*III), followed by dividing the digested beaded-cDNA into two equal parts. Two specific likers, DNA liker-A and liker-B with sticky overhands, are added for the ligation reaction. These linkers have built in the recognition motif for the tagging enzyme, usually *Bsm*FI, a type IIs enzyme. This enzyme has the ability to cut the cDNA 9-12 bp 3' from the recognition. These short fragments are used to identify the cDNA types. Mix two aliquots and ligate these tags tail to tail. PCR amplification is performed using primers with the sequences identical to the parts of the sequence in liker-A and -B. Each PCR fragment contains a ditag which realeased from the linkers by the above 4-bp restriction enzyme. These ditags are concatenated, cloned, and sequenced. Each clone contains 10 to 50 tags that are divided by a four-base interval. The abundance of each tag in the cloned products is directly proportional to the abundance of the corresponding transcript in the original sample. The relative abundance of a transcript is calculated by dividing each specific tag count by the total number of tags. However, this analysis method was very time consuming and labor intensive and it was difficult to get expression profilings of multiple samples for given genes at a time. Also it is necessary to own a sequencer in the lab for SAGE analysis, and a regular lab cannot afford an expensive sequencer.

DNA microarray is a method using cDNAs or oligonucleotides on solid matrices to hybridize the labeled cDNAs transcripted from mRNA. The readout of expressed genes by this technique relies on comparative hybridization with probes from two kinds of sample, which are labeled with different dyes. The relative gene expression levels are obtained by comparing the signal intensities by two different dyes in each spot on the microarray. It requires expensive hardware for arraying, scanning, and analysis of the experiments.

A review of methods for gene expression profiling was described in Nature Biotechnology, 14, 1675-1680 (1996).

Pyrosequencing is a new developed technology for real-time DNA sequencing based on bioluminometric assay [Science, 281, 363, 365 (1998); Anal Biochem, 280,103-10 (2000)]. As only 10-30 bases can be sequenced at a time, it mainly applied for SNP detection. This method relies on quantitative PPi detection for sequencing, and has many advantages such as the ability of quantitative analysis, high sensitivity, easy processing, no electrophoresis, no requirement for a labeling reaction or a laser source, no use of special or expensive reagents, as well as simple automation. Pyrosequencing employs coupled enzymatic reactions for monitoring the PPi produced by the sequencing reaction. PPi in a quantity equimolar to the amount of incorporated nucleotide was released by polymerase-catalyzed extension reaction if the added dNTP is complementary to the base in the template strand. ATP sulfurylase quantitatively converts PPi to ATP in the presence of adenosine 5' phosphosulfate (APS). The produced ATP drives the luciferase-catalyzed light production reaction under the substrate of luciferin. The visible light is detected by a charge-coupled device (CCD) camera or photomultiplier tube (PMT) and seen as a peak in the sequencing profiles. As the signal intensities are proportional to the amount of PPi, the DNA sequence is obtained by the species of the incorporated dNTP and the relative peak intensities.

However the technique cannot be used for gene expression profiling directly.

### SUMMARY OF THE INVENTION

In addition to the drawbacks described above for the gene expression profiling methods, conventional electrophoresis-based expression profiling techniques for comparing gene expression level between different sources has the problem that the ratio between PCR products does not accurately reflect the ratio of the quantities of cDNA templates between various sources. The purpose of the present invention is to provide an accurate and sensitive method for comparing the difference in expression profile between plural samples in one tube by the processes on the basis of pyrosequencing.

The inventive method for assaying gene expression levels in a mixture by sequencing comprises:
step 1 of labeling the mRNA of a given gene from different sources, respectively, through a suitable labeling process;
step 2 of mixing the labeled fragments in one receptacle as PCR templates;
step 3 of performing PCR by using source-specific oligonucleotides and a gene-specific oligonucleotide as primers; and
step 4 of detecting the sequence of the amplified DNA fragments by the method based on bioluminometric assay.

The inventive method for comparing gene expression profiling at various sources is characteristic as follows.
1. The different sources mean a given gene from different individuals, or a given gene from different organs, or a given gene at different states.
2. The source-specific primers comprise the identical base species and the identical base number, but the different base order; and each of the source-specific primer characterizes one gene source.
3. The suitable labeling process means the method using different DNA fragments for characterizing gene sources. There are three ways for the labeling. The first way is to perform a reverse transcription-polymerase chain reaction (RT-PCR) to obtain complementary DNA (cDNA) fragments of a given gene in each of the sources, followed by the digestion with a restriction enzyme. Finally each of the digested cDNA fragments is ligated to a corresponding DNA adapter that has a known nucleotide sequences. The second way is to synthesize the first strand of the complementary DNA (cDNA) fragments of mRNA samples from each of the sources using polythymine primers fixed on microsphere's surface, and the corresponding second strand is synthesized using source-specific anchored primers containing the sequences for identifying the gene sources in the 5'-terminal region. The third method is to use the source-specific anchored primers containing the sequences for identifying the gene sources in the 5'-terminal region to synthesize the first cDNA strand directly.
4. The adapters comprise different nucleotide sequences, but with the same nucleotide composition. Each of the DNA adapters corresponds to each of the gene sources;
5. The source-specific anchored primers are composed of the same length and the same composition of nucleotides, but have the different base sequences.

There are two key points for this invention. The first one is to amplify plural cDNAs in one tube using source-specific primers having the same melting temperature, and the ratio of the quantities among the amplified fragments can accurately reflect the ratio of the quantities among the corresponding templates. The second one is to use the base sequence in the sequencing profile to identify the gene source, and to use the signal intensity of each corresponding base to quantify gene expression levels of various sources.

The first key point is realized by ligating the digested dsDNA fragments with source-specific adapters being composed of oligomers having same melting temperature, or by synthesizing cDNAs using anchored primers having source-specific tags in the 5'-terminus. The source-specific sequences have the same composition of nucleotides but the different base-order, so that the primers for the following PCR amplification have the same meting temperature. The second key point is realized by introducing a short sequence having the identical base species but the different base-order into source-specific adapters or primers. Each of source-specific PCR products has a unique base sequence so that the gene-source and the corresponding expression levels can be deduced from the base species and its intensity in the sequencing profiles respectively.

By the method of the present invention, gene expression profiling of a given gene at different sources can be carried out at a time. As it is based on bioluminometric assay, the advantages include high sensitivity, low running cost, accurate quantification and simple operation.

### BRIEF DESCRIPTION OF THE DRAWINGS

FIG 1 illustrates a procedure to detect the difference of gene expression level between various sources according to the present invention which is precisely figured out in Embodiment 1;
FIG 2 is a schematic diagram illustrating the procedure for detecting the difference of the expressed level of a given gene in two different sources which is described in Embodiment 1;
FIG 3 depicts the structure of DNA adapters of Embodiment 1 in accordance with the invention;
FIG 4 shows the reaction module for detecting the sequencing of PCR amplified products by bioluminometric method in Embodiment 1 of the invention;
FIG 5 shows the sequencing profile for comparing the gene expression levels from two sources by using real-time bioluminometric assay in Embodiment 1 of the invention;
FIG 6 depicts a schematic structure of the device using a 96-well plate for detecting expression levels of plural genes from various sources in Embodiment 2 of the invention;
FIG 7 shows a procedure of gene expression profiling by using two pooled samples in Embodiment 4 of the invention; and
FIG 8 shows the sequencing results of the sample in Embodiment 1 of the invention by using the pyrophosphate (PPi) detection solution without apyrase, which is described in Embodiment 5.

### DESCRIPTION OF THE PREFERRED EMBODIMENTS

The followings are the embodiments of the present invention.

### EMBODIMENT 1

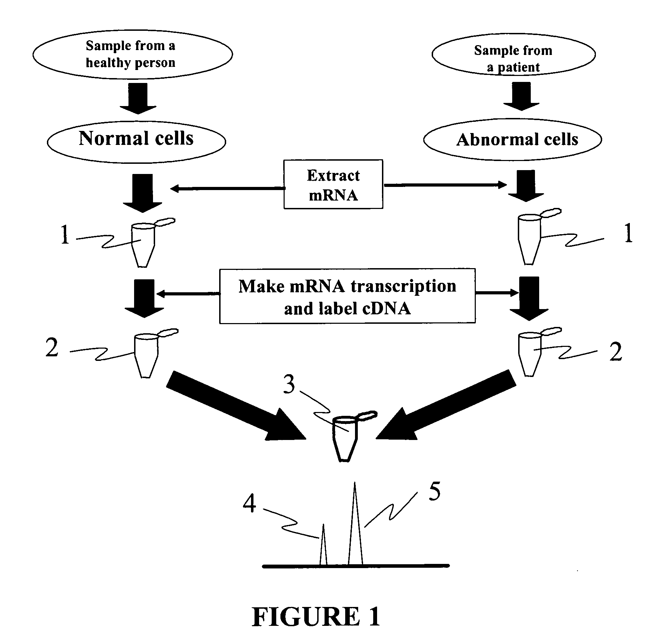

The flowchart of comparing the gene expression levels between various sources is illustrated in FIG 1. The sample cDNAs are prepared from the RNAs extracted from normal cells of a healthy person and abnormal cells of a patient respectively. The extracted RNAs are converted to cDNAs 1 by using reverse transcriptase, and cDNAs from each of sources are labeled with source-specific sequences respectively. By subsequently mixing the labeled cDNA fragments 2 in one tube as template DNA fragment for PCR, amplified DNA fragments 3 are obtained by source-specific primers and a gene-specific primer. Finally the PCR products are sequenced by bioluminometric assay. The gene-sources of normal cells and abnormal cells are identified as peak 4 and peak 5 respectively, and the corresponding expression levels are determined from the intensity of each of the peaks in the sequencing profiles.

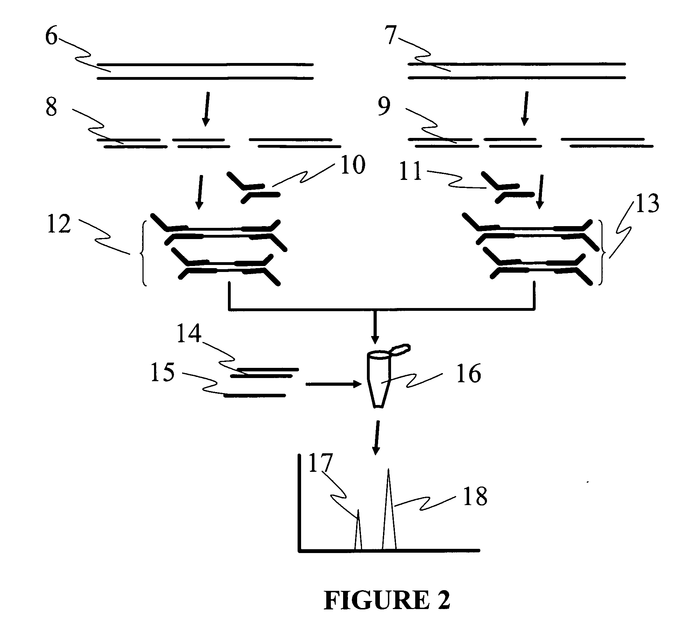

FIG 2 is a schematic diagram illustrating the procedure for detecting the difference of the expressed quantity of a given gene in two different sources. As shown in FIG 2, sample cDNA 6 from source A and sample cDNA 7 from source B (in a mixture of various cDNAs; one cDNA is shown in the figure) are digested with a restriction enzyme to prepare cDNA fragments 8 and 9 respectively. By ligating oligomers (namely DNA adapter) 10 with the cDNA fragment 8, cDNA fragments 12 with the DNA adapter 10 are produced. By ligating oligomers (DNA adapter) 11 with the cDNA fragment 9, cDNA fragments 13 with the DNA adapter 11 are produced. By subsequently mixing together the cDNA fragment 12 and the cDNA fragment 13 to prepare a mixture, a part of the solution is used as template DNA fragments of PCR. PCR amplification is performed in tube 16 by adding two source-specific primers 14 and a gene-specific primer 15. The amplified DNAs are sequenced by incorporating nucleotides step-by-step, and the peak 17 as well as peak 18 are corresponding to the relative gene expression levels in source A and source B respectively.

Here the amount of expressed P53 gene in source A and B is compared. At first, cDNAs from each of sources are prepared separately. The extraction of mRNA is carried out by using Gibico TRI20LLS-Reagent™ kit. The first strand of cDNAs is synthesized using the kit of Gibico Super Script™ Preamplification System for First Strand cDNA Synthesis. The second strand cDNA is synthesized by adding 1 µl of 1 mM dNTPs mixture, 50 U polymerase I, 10 U DNA ligase, 2U RNase H, 10 µ of 10× ligation buffer and 70 µl H₂O into 20 µl of the first strand cDNA solution, and the mixture is incubated at 10 °C for 2 hours and 70 °C for 15 min. Enzymatic digestion is performed by adding 60 U restriction enzyme Mbo I, 20 µl of 10× digestion buffer (200 mM Tris-HCl (pH8.5), 100 mM MgCl₂, 10 mM dithiothreitol (DTT) and 1 M KCl) and 30 µl H₂O into 150 µl of the above second strand cDNA solutions, and the mixture is incubated at 37 °C over night and 70 °C for 15 min.

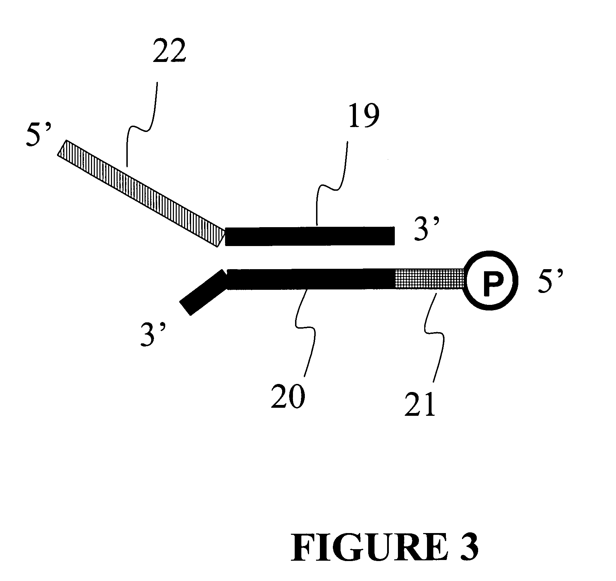

Secondly, DNA adapters are prepared to ligate with the above digested cDNA fragments for identifying the gene sources. FIG. 3 depicts the structure of DNA adapter. The DNA adapter is composed of two partly complementary strands of oligonucleotide 19 and oligonucleotide 20. In order to ligate with the digested cDNA fragment, 5' terminus of oligonucleotide 20 of each adapter contains a sequence 21, a restriction enzyme recognition nucleotide sequence GATC. To avoid the extension reaction, the sequence of the 3' terminus of oligonucleotide 20 is not complementary to strand 19. In the 5' terminus of oligonucleotide 19, there is a sequence 22 for identifying the gene source. Each DNA adapter recognizes only one gene source. The base order of sequence 22 in each of DNA adapters is different, but sequence 22 is composed of the same nucleotide species. All of the sequences except for sequence 22 in DNA adapters are identical. For labeling gene source A and source B, two types of oligonucleotide 19, namely P-1 (SQ ID No. 1) and P-2 (SQ ID No. 2), as well as a common sequence oligonucleotide 20, namely P-3 (SQ ID No. 3), are prepared.

SQ ID No. 1

5’-CCCCACTTCTTGTTCTCTCATCAGGCGCATCACTCG-3’

SQ ID No. 2

5'-CACCTCTCATTTCTCCCTGTTGACGCGCATCACTCG-3'

SQ ID No. 3

5'-GATCCGAGTGATGCGCTAAG-3'

For ligation reaction, 10 pmol P-1 and 10 pmol P-3 are added into a tube containing the digested cDNA solution of source A, and 10 pmol P-2 and 10 pmol P-3 are added into another tube containing the digested cDNA solution of source B. Each of two ligation solutions contains 25 mM Tris-HCl (pH7.6), 6.5 mM MgCl₂, 0.5 mM ATP, 0.5 mM DDT and 2.5% polyethene glycol-800. The mixture is incubated at 70 °C for 10 min and slowly cooled down to 16 °C. After adding T4 DNA ligase, ligation reaction is performed at 16 °C for 2 hours. These ligation products are mixed together as the templates for the following PCR.

Thirdly, PCR amplification of the DNA adapter-labeled cDNA fragments is performed using the mixed ligation solution as templates in one reaction tube. The source-specific primers, namely MP-1 (SQ ID No. 4) and MP-2 (SQ ID No. 5), are the oligonucleotides having the same sequences as the first 21 bases of P-1 and P-2 from 5' terminus, respectively. The other primer for PCR is P53 gene-specific oligonucleotide, namely GSP (SQ ID No. 6). For preparing single stranded DNA, 5' terminus of GSP is modified by biotin.

SQ ID No. 4

5'-CCCCACTTCTTGTTCTCTCAT-3'

SQ ID No. 5

5'-CACCTCTCATTTCTCCCTGTT-3'

SQ ID No. 6

5'- biotin-GGAGCACTA AGCGAGCACTG-3'

PCR solution contains 20 mM Tris-HCl pH 8.0, 50 mM KCl, 0.2 mM each of dNTPs, 1.25 U DNA polymerase, 1 pmol MP-1, 1 pmol MP-2, 1 pmol GSP and 1 µl of templates. PCR reaction is carried out at the thermal cycling conditions of 30 cycles at 94°C for 30 s, 58°C for 1 min and 72°C for 30 s. The double stranded biotin-PCR products are combined with streptavidin-coated beads (Dynabeads M280) at room temperature for 30 min in the buffer of 5 mM Tris-HCl pH7.5, 0.5 mM EDTA and 1.0 M NaCl. After the reaction, single stranded DNA is prepared by adding 0.1 M NaOH for the incubation at room temperature for 5 min. The beads are then collected and washed by the buffer of 5 mM Tris-HCl pH7.5, 0.5 mM EDTA and 1.0 M NaCl. Single stranded DNAs from both sources are contained in the surface of the beads.

Fourthly, single stranded DNAs prehybridized with primers MP-1 and MP-2 are sequenced by bioluminometric assay. The solution for bioluminometric assay contains 0.1M Tris-acetate (pH7.7), 2mM EDTA, 10mM magnesium acetate, 0.1% bovine serum albumin (BSA), 1mM dithiothreitol (DTT), 3µM adenosine 5'-phosphosulfate (APS), 0.4mg/ml polyvinylpyrrolidone (PVP), 0.4mM D-luciferin, 200 µU/ml ATP sulfurylase, luciferase in an amount giving a suitable sensitivity, 20 U/ml apyrase and ploymerase. The sequencing reactions start by dispensing dGTP and dCTP respectively. In stead of dGTP and dCTP, ddGTP and ddCTP, or their analogues can be also used for the extension reaction. The signal intensity obtained by adding dGTP represents the relative expression level of P53 gene in source A. The signal intensity obtained by adding dCTP represents the relative expression level of P53 gene in source B.

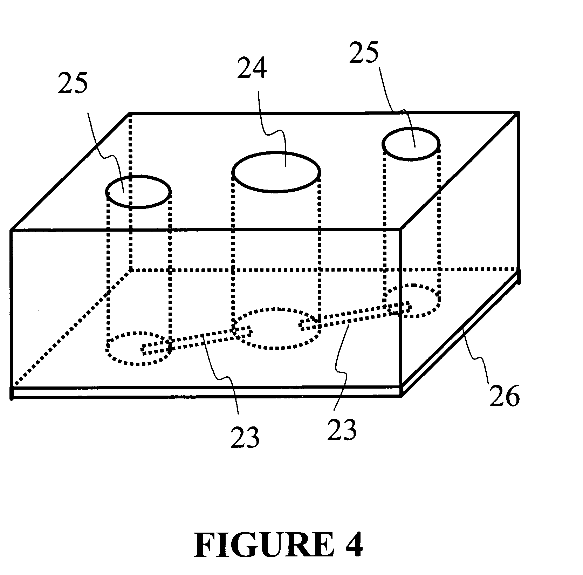

FIG 4 shows the reaction module for detecting the sequence of PCR amplified products by bioluminometric method. Capillaries 23 are used for connecting reaction chamber 24 and dNTP reservoir 25. The flow of dNTP from reservoir 25 to the reaction chamber 24 starts by adding a pressure on the reservoir 25. The light released from the extension reaction goes through a transparent slide and is detected by a light sensor such as a charge-coupled device (CCD) camera and a photomultiplier tube (PMT).

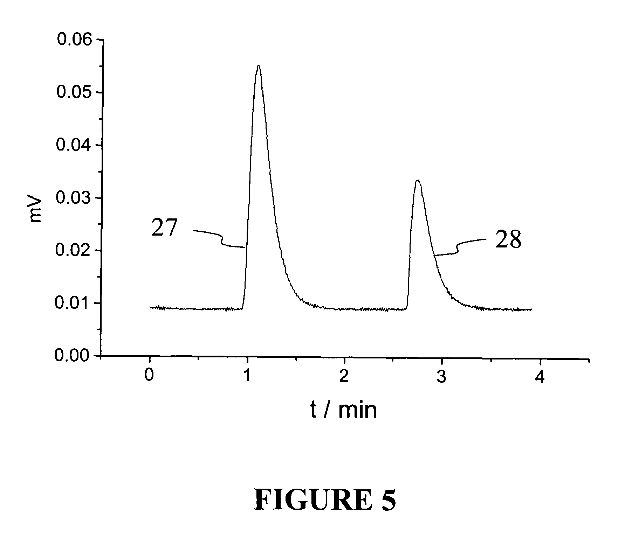

FIG. 5 is the sequencing profile for comparing the expression levels of P53 between source A and B by using real-time bioluminometric assay. Peak 27 is the signal produced by adding dGTP for the sequencing reaction, and it represents the amount of the expressed P53 gene in source A. Peak 28 is the signal produced by adding dCTP for the sequencing reaction, and it represents the amount of the expressed P53 gene in source B. The relative expression levels of P53 gene in these two sources can be calculated from these two signal intensities.

### EMBODIMENT 2

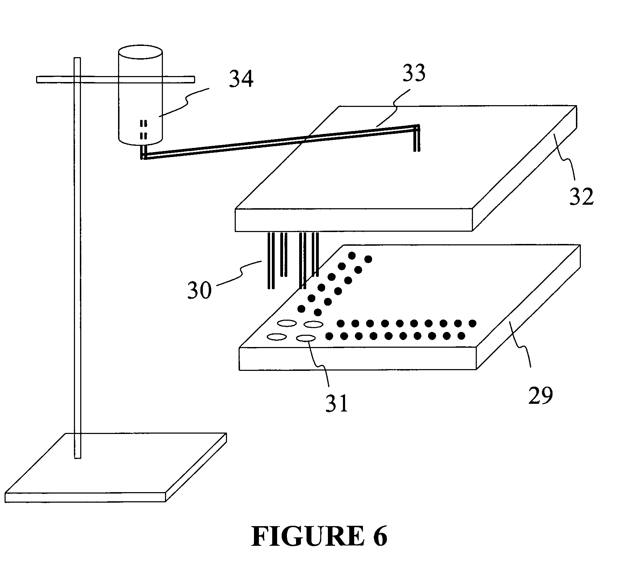

In this embodiment, 96-well plate is used for detecting the gene expression levels of 96 samples simultaneously. The sample preparation is the same as that in embodiment 1. The key point of this example is the device for detecting 96 samples in parallel. FIG 6 depicts a schematic structure of the device using a 96-well plate for detecting expression levels of plural genes from various sources. According to the dimension of 96-well plate 29, liquid dispensers are made by using capillaries 30 to add dNTP to wells 31 of plate 29. Capillaries 30 are connected with the plate 32 which has a chamber to hold dNTP solution. A tube 33 is used to transfer dNTP solution from dNTP reservoir 34 to the plate 32. When adding pressure in dNTP reservoir 34, dNTP solution flows into wells 31 to trigger the extension reaction. PPi released during the dNTP incorporation is quantitatively converted to ATP in the presence of APS under the catalysis of ATP sulfurylase. The produced ATP drives the light production in the presence of luciferin and luciferase. The light intensity is proportional to the amount of DNA template. A charge-coupled device (CCD) camera, PMT or photodiode array can be used to detect the signal released from the wells 31. When adding another dNTP for the sequencing reaction, the same dNTP dispenser is required. Here two kinds of dNTP, dGTP and dTTP, are dispensed into the plate 29 in turn, therefore two identical dispensers are built as the same way. This device can be used for detecting multiple samples simultaneously.

### EMBODIMENT 3

In this embodiment, comparison of the expression level of one given gene among six different sources is performed by six different adapters having known different sequences. As dATP is an analog of ATP, it produces high background signals. Although an analog of dATP, dATPαS, can be used for the detection, it is very expensive. In this invention, only three kinds of dNTP are used for sequencing. Consequently, only three sources can be compared simultaneously if one dNTP represents one source. As bioluminescent assay of PPi has a very good quantitative capability, a large range of PPi can be determined linearly. Here the six adapters have the same structures as that in FIG 3 except for the sequence of oligonucleotide 19. For labeling six gene sources, six types of oligonucleotide 19, namely P-3 (SQ ID No. 7), P-4 (SQ ID No. 8), P-5 (SQ ID No. 9), P-6 (SQ ID No. 10), P-7 (SQ ID No. 11) and P-8 (SQ ID No. 12) are designed.

SQ ID No. 7

5'-CCC CAC TTC TTG TTC TCT CAT CTGA CGCATCACTCG-3'

SQ ID No. 8

5’-CAC CTC TCA TTT CTC CCT GTT GTCA CGCATCACTCG-3’

SQ ID No. 9

5'-CCC CAT CAC TTC TTC TTG TCT AGCT CGCATCACTCG-3'

SQ ID No. 10

5'-CAC TTT TCA CCT CTC GTT CTC GACT CGCATCACTCG-3'

SQ ID No. 11

5’- TTG CAT CAC TTC TTC TCT CCC CAGT CGCATCACTCG-3’

SQ ID No. 12

5'- TTT CAC GTT TCA CTC CTC CCT ATCG CGCATCACTCG-3'

Ligate cDNA from each of gene sources with each of six DNA adapters separately. Mix the ligation solution in one reaction tube, and part of this mixture is used as the templates of PCR. PCR amplification is performed using the source-specific primers and a gene-specific primer. The source-specific primers, namely MP-3 (SQ ID No. 13), MP-4 (SQ ID No. 14), MP-5 (SQ ID No. 15), MP-6 (SQ ID No. 16), MP-7 (SQ ID No. 17) and MP-8 (SQ ID No. 18), are the oligonucleotides having the same sequences as the first 21 bases of P-3, P-4, P-5, P-6, P-7 and P-8 from the 5' terminus, respectively. P53 gene-specific primer is GSP (SQ ID No. 6).

SQ ID No. 13

5'-CCC CAC TTC TTG TTC TCT CAT-3'

SQ ID No. 14

5'-CAC CTC TCA TTT CTC CCT GTT -3'

SQ ID No. 15

5’-CCC CAT CAC TTC TTC TTG TCT -3’

SQ ID No. 16

5’-CAC TTT TCA CCT CTC GTT CTC -3’

SQ ID No. 17

5'- TTG CAT CAC TTC TTC TCT CCC -3'

SQ ID No. 18

5'- TTT CAC GTT TCA CTC CTC CCT -3'

Single stranded DNAs are prepared by the same method in embodiment 1. After hybridized with the mixtures of primers MP-3, MP-4, MP-5, MP-6, MP-7 and MP-8, the single stranded DNAs are sequenced by adding dTTP, dGTP, dCTP, dTTP, dGTP and dCTP respectively. Six peaks appear in the sequencing profile. If the sequences of P-3, P-4, P-5, P-6, P-7 and P-8 represent sources A, B, C, D, E and F respectively, the first peak in the sequencing profile reflect the gene expression levels of source C plus source F. The second, the third, the fourth, the fifth and the sixth peaks reflect the gene expression levels of source A plus source E, source B plus source C plus source D, source D plus source E, source C plus source D, and source E respectively. The relative gene expression level from each of sources can be calculated readily by simultaneous equations.

### EMBODIMENT 4

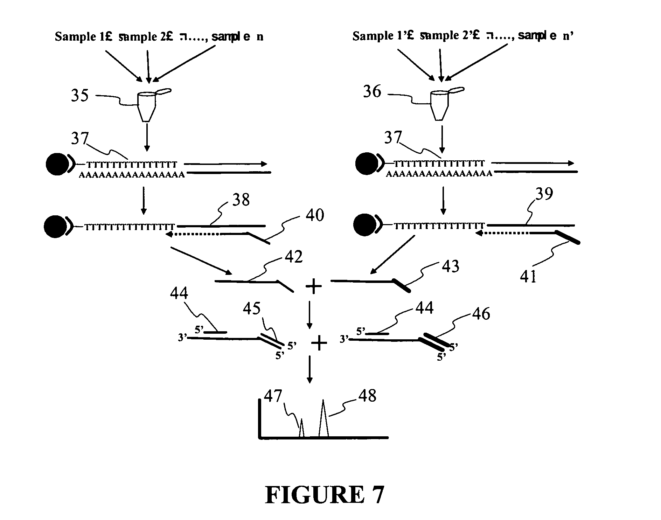

In this embodiment, comparison of average gene expression level between pooled samples is described. Usually, gene expression profiling is carried out using many individuals so that an accurate result can be obtained. If we want to evaluate a gene expression profiling between two groups, and each of groups contains 100 cases, at least 100 detections are required by microarray assay. If we pool 100 samples as one sample, only two detections are needed for comparing the gene expression level between the two pooled samples. The procedure for the detection is described in FIG. 7. At first, sample 1, 2, ......, n in one group are pooled in tube 35, and then sample 1', 2', ......, n' in another group are pooled in tube 36. The first strand cDNAs 38 and 39 are synthesized using beaded primer 37. The source-specific anchored primer 40 and 41 are used to synthesize the second strands 42 and 43 respectively. Mix equally the second strands 42 and 43 in one tube. PCR amplification is performed by adding a gene-specific primer 44 as well as source-specific primers 45 and 46 in one tube. After the preparation of the single stranded PCR products, sequence reaction is carried out by bioluminescent assay. The relative intensities of peak 47 and 48 reflect the difference of gene expression level between two pooled samples. The advantage for this strategy is the high efficiency of the detection.

### EMBODIMENT 5

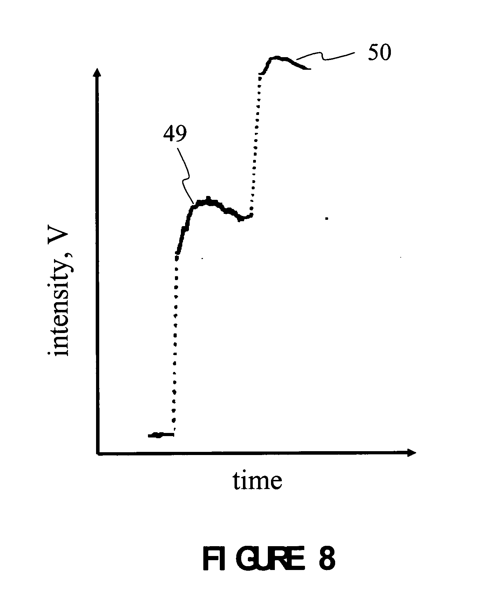

In pyrosequencing, an enzyme of apyrase is employed to degrade dNTP and ATP for the successive sequencing reaction. However it is not necessary to degrade dNTP in the present invention when comparing the gene expression levels between two or three sources, because the excess dNTP from the previous dNTP dispensing does not affect the next dNTP extension reaction. In addition, it is not necessary to degrade ATP to get a peak in the sequencing profile as the signal intensities produced by ATP can be easily controlled in a linear range. The same sample in embodiment 1 is detected by the bioluminescent assay without the use of apyrase. The results are showed in FIG 8, and signal 49 and signal 50 are produced by adding dGTP and dCTP respectively. The signal intensities are the same as those obtained by using apyrase.

## Claims

1. A method for comparatively analyzing gene expression level from different sources comprising:
(1) labeling mRNA from different sources, respectively, using a suitable method; and then mixing the labeled fragments equally as the templates of polymerase chain reaction (PCR); and
(2) performing a polymerase chain reaction to obtain amplified DNA fragments, in one receptacle, using source-specific primers and a gene-specific primer for the amplification; and
(3) detecting the sequence of the amplified DNA fragments by the method based on bioluminometric assay, and the base type and the signal intensity in the sequencing profile representing the gene source and the relative expression level respectively.

2. The method of claim 1 wherein mRNA from different sources is the expressed mRNA of a given gene from different individuals, or is the expressed mRNA of a given gene from different organs of a individual, or is the expressed mRNA of a given gene of the same species at different states of chemical stimulation or physical stimulation.

3. The method of claim 1 wherein the source-specific primers comprise the identical base species and the identical base number, but the different base order.

4. The method of claim 1 wherein a suitable method is the way to distinguish the gene source by a DNA fragment; wherein the way is:
to distinguish the gene sources by performing a reverse transcription-polymerase chain reaction (RT-PCR) to obtain complementary DNA (cDNA) fragments of a given gene in each of the sources, followed by the digestion with a restriction enzyme, and then ligating each of the digested cDNA fragments with a source-specific DNA adapter;
to distinguish the gene sources by synthesizing the first strand of the complementary DNA (cDNA) fragments of mRNA samples from each of the sources using polythymine primers fixed on microsphere's surface, and then synthesizing the corresponding second strand using source-specific anchored primers containing the sequences for identifying the gene sources in the 5'-terminal region; and
to distinguish the gene sources by directly synthesizing the first strand of the complementary DNA (cDNA) fragments of mRNA samples from each of the sources using the source-specific anchored primers containing the sequences for identifying the gene sources in the 5'-terminal region.

5. The method of claim 4 wherein a source-specific DNA adapter contains a part of sequences complementary to the recognition sequences of the restriction endonuclease used for digesting cDNA fragments, and is ligated with the digested cDNA fragments by a DNA ligase; wherein:
each of the source-specific DNA adapters comprises two strands of "Y" shape, and one of the strands contains source-specific sequences in the 5'-end region, and the other strand has a no-extension 3'-end; wherein the no-extension 3'-end:
has a sequence non-complementary to the other strand in the 3'-end region;
contains a modified base in the 3' terminus, and the modified base lacks the ability of polymerase extension reaction.

6. The method of claim 4 wherein the source-specific DNA adapters for identifying each of gene sources comprise identical base species and an identical base number, but the different base order; and the method of claim 4 wherein the source-specific anchored primers for identifying each of gene sources comprise identical base species and an identical base number, but the different base order; wherein:
each of source-specific DNA adapters has the same melting temperature; and
each of source-specific anchored primers has the same melting temperature.

7. The method of claim 1 wherein bioluminometric assay is based on the quantitative determination of pyrophosphate released from the polymerase extension reaction.

8. The method of claim 7 wherein the extension reaction is the polymerization of the single-stranded PCT products annealed with a given primer or primer mixtures; and the polymerization starts by adding a kind of deoxynucleotide (dNTP)s, or a kind of dideoxynecleoide (ddNTP)s, or a kind of analogues of dNTPs or ddNTPs, respectively, in a given order.

9. The method of claim 8 wherein the single stranded PCR products are obtained by treating PCR products (described in claim 1) by using the physical method or the chemical method; wherein:
the physical method is to use biotinlated primer for PCR amplification and then preparing the single-stranded DNAs by a solid phase method; and
the chemical method is to use a kind of enzymes for the digestion to prepare single strand.

10. The method of claim 7 wherein the extension reaction is to use single-strand binding protein (SSB) for the polymerization of the PCT products annealed with a given primer or primer mixtures; and the polymerization starts by adding a kind of deoxynucleotide (dNTP)s, or a kind of dideoxynecleoide (ddNTP)s, or a kind of analogues of dNTPs or ddNTPs, respectively, in a given order; wherein the PCR products:
are treated by enzymes to remove PPi produced during PCR reaction, the excess dNTPs as well as the excess primers, prior to the annealing process.
